# DEMANDE DE BREVET EUROPEEN

(11) **EP 0 670 151 A2**
(43) Date de publication de la demande: **06.09.1995**
(21) Numéro de dépôt: 95400035.2
(22) Date de dépôt: 09.01.1995
(51) Int. Cl.: A61F 2/38

(54) **Prothèse de genou**

(30) Priorité: 10.01.1994 FR 9400149
(71) Demandeur: Billy, Jean-Louis, F-87270 Bonnac-la-Côte (FR)
(72) Inventeur: Billy, Jean-Louis, F-87270 Bonnac-la-Côte (FR); Duquet, Bruno, F-87000 Limoges (FR); Chataignier, Jean-Jacques, F-44000 Nantes (FR); Tubiana, Didier, F-91450 Soisy-sur-Seine (FR); Lefèvre, Christian, F-29000 Brest (FR); Labbe, Jean-Claude, F-87000 Limoges (FR)
(74) Mandataire: Laget, Jean-Loup

(57) **Abrégé**

La prothèse de genou comprend une pièce fémorale (1) reliée à un plateau tibial (3) par une plaque méniscale intermédiaire (2), la pièce fémorale (1) étant composée d'un bouclier trochléen (5) et d'une plaque fémorale (7) reliée à la partie inférieure du bouclier trochléen (5) et s'étendant vers l'arrière. Les faces externes des parties latérales (10) de la pièce fémorale (1) sont cylindriques et forment des condyles coopérant avec des portées de guidage cylindriques (13) ménagées dans la face supérieure de la plaque méniscale (2). La face inférieure (15) de celle-ci est convexe et sphérique afin de coopérer avec la face supérieure concave et sphérique (24) du plateau tibial (3). Toutes les surfaces de frottement sont au moins partiellement recouvertes d'une céramique encastrée qui fait légèrement saillie par rapport à la surface de frottement.

## Description

L'invention concerne une prothèse de genou tricompartimentale ainsi qu'une prothèse rotulienne associée à celle-ci.

Il existe déjà sur le marché de nombreux dispositifs de ce type, mais à la connaissance du demandeur, jusqu'à ce jour, aucune de ces prothèses n'a pu répondre à l'usure des pièces de frottement en contact, usure des plateaux en polyéthylène par la pièce fémorale en métal et cette usure va en s'accélérant avec le temps. Les prothèses connues sont toutes à cinétique de destruction relativement rapide, ce qui à ce jour engendre des réinterventions chirurgicales lourdes.

En général, les fabricants de prothèses ont tenté de reproduire aussi fidèlement que possible l'articulation concernée, tant dans ses dimensions physiques que dans son fonctionnement : répartition des charges, axe physiologique du mouvement etc.

Toutefois les concepteurs et réalisateurs de prothèses de genou ont depuis longtemps composé avec les matériaux qui étaient à leur disposition lors de la création de la présente invention.

L'articulation "naturelle" du genou possède deux surfaces de glissement souples, à savoir les ménisques, qui ont une fonction fondamentale dans ce type d'articulation.

D'où l'idée pour les prothèses d'articulation de mettre en oeuvre des pièces de substitution mobiles. Depuis les années 1960, de telles pièces mobiles intermédiaires sont utilisées pour les arthroplasties.

Parmi les prothèses de genou, on peut citer la prothèse décrite dans le brevet français 2 290 883 et la prothèse décrite dans le brevet européen 0 021 421.

Ces prothèses mettent en oeuvre des pièces intermédiaires mobiles. La prothèse du premier brevet est unicompartimentale. Elle ne règle donc que quelques cas de lésion du genou et ce n'est pas une prothèse qui s'adapte aux lésions de gravité croissante. Certes, elle peut être posée en prothèse bicompartimentale, toutefois il est à noter que cette prothèse est dépourvue de bouclier trochléen pour faire face à une prothèse rotulienne.

De plus elle ne peut être posée qu'en présence d'un ligament croisé antérieur intact et de ligaments latéraux non distendus. Il est par ailleurs impossible d'effectuer un ajustement une fois les coupes faites, si ce n'est de faire varier l'épaisseur des pièces mobiles. Enfin, elle doit être obligatoirement ancrée à l'os par un ciment.

La deuxième prothèse citée présente, elle aussi, un certain nombre d'inconvenients, entre autres :
- Lors de la pose en prothèse unicompartimentale, le principe du rail sur l'implant tibial impose un positionnement extrêmement précis de la pièce fémorale, alors que celle-ci n'est pas déterminable car elle est fonction de la forme de l'os, mais surtout de la tension ligamentaire.
- Il n'y a pas de réglage possible une fois les coupes faites, si ce n'est de modifier l'épaisseur de la pièce mobile.
- Lors de la pose en unicompartimentale, la présence du ligament croisé anterieur est indispensable.
- Le ou les coulisseaux qui glissent dans le rail curviligne et qui y maintiennent le patin mobile en PEHD constituent une structure fragile peu compatible avec la longévité attendue du produit
- Lors du recul des pièces mobiles par flexion, si le ligament croisé antérieur est absent, aucune force de rappel n'agit ni ne freine les pièces mobiles si bien qu'en flexion, les condyles fémoraux vont prendre avec l'usure une position de plus en plus reculée.
- Lors de la flexion, les pièces mobiles se déplacent en arrière mais aussi se rapprochent. Cette position amène une diminution des surfaces en contact et donc une surcharge pondérale très importante condyles-pièces mobiles, ce qui aggrave la situation précédente.
- Enfin, ces deux prothèses comme l'ensemble des prothèses créées jusqu'à ce jour ne permettent pas un auto-positionnement corrigeant le manque de precision des coupes et la destruction des pièces mobiles est alors entraînée par le manque de degrés de liberté qui ne permet pas un positionnement correct de la prothèse.

Une prothèse de genou plus récente d'un autre type est connue du EP-A 186 471. Cette prothèse est pourvue d'une plaque méniscale dont la face inférieure est conique et coopère avec une face également conique d'un plateau tibial. Cette agencement apporte une certaine amélioration par rapport aux autres prothèses antérieurement mentionnées, mais elle manque de flexibilité naturelle.

La présente invention a pour but de pallier l'ensemble des inconvénients tels que décrits ci-dessus et concerne une prothèse destinée à être utilisée dans le domaine de la chirurgie orthopédique pour remplacer les surfaces de glissement de l'articulation du genou, usées ou détruites lors de l'arthrose, d'arthrite, rhumatoïde, ou de fractures complexes femoro-tibio-patellaires.

L'invention concerne également une prothèse rotulienne associée à la prothèse de genou suivant l'invention. Des prothèses rotuliennes sont décrites dans par exemple le US-A 4 007 495 et le DE-U 8 325 767. Ces prothèses présentent l'inconvénient qu'elles laissent très peu de liberté de mouvement à la rotule, ce qui est incompatible avec un bon fonctionnement aussi naturel qui possible de cette pièce.

L'invention a pour objet une prothèse de genou comprenant une pièce fémorale reliée à un plateau tibial par une plaque méniscale intermédiaire, la pièce fémorale étant composée d'un bouclier trochléen et d'une plaque fémorale reliée à la partie inférieure du bouclier trochléen et s'étendant vers l'arrière, les faces externes des parties latérales de la pièce fémorale étant cylindriques et formant des condyles coopérant avec des portées de guidage cylindriques ménagées dans la face supérieure de la plaque méniscale, la face inférieure de celle-ci étant convexe afin de coopérer avec la face supérieure concave du plateau tibial, caractérisée en ce que toutes les surfaces en contact de frottement les unes avec les autres sont au moins partiellement pourvues d'une céramique encastrée dans lesdites surfaces de frottement.

Selon d'autres caractéristiques de l'invention:
- les céramiques font légèrement saillie par rapport aux surfaces de frottement dans lesquelles elles se trouvent encastrées;
- l'épaisseur de la céramique encastrée est comprise entre 0,1 et 0,6 mm;
- l'une de deux surfaces de frottement coopérant l'une avec l'autre est partiellement recouverte par des céramiques encastrées sous forme de bandes.

La prothèse suivant l'invention abandonne par ailleurs l'imitation des formes anatomique et permet de reproduire la mécanique cinétique normale du genou et le but est atteint grâce à une pièce intermédiaire mobile formant ménisques artificiels, qui permettent d'une part un roulement des condyles de la pièces fémorale et d'autre part un glissement de la pièce mobile sur le plateau tibial.

Le glissement de la pièce intermédiaire sur le plateau tibial présente une infinité de degrés de liberté de mouvement obtenus par l'ajustement des deux portions de sphères.

Ce système permet l'auto-positionnement de l'ensemble des trois pièces et compense automatiquement les défauts de coupes osseuses. Le roulement-glissement est dosé par l'appareil ligamentaire lui-même. L'insuffisance ou l'absence du ligament croisé antérieur ne s'oppose pas à la mise en place de la prothèse. Ce manque ou insuffisance sont compensés par l'équilibre cinématique dû aux formes mécaniques de la prothèse.

Selon encore d'autres caractéristiques de l'invention:
- les faces convexe et concave de la plaque méniscale et du plateau tibial, respectivement, sont sphériques, et le centre des portées cylindriques de la pièce fémorale est disposé, lorsque le genou est en position d'extension verticale, dans un plan vertical décalé par rapport à un plan vertical passant par le centre de la portée sphérique du plateau tibial, de manière à provoquer un glissement de la plaque méniscale sur le plateau tibial d'arrière en avant lors de la flexion du genou;
- le rayon de la sphère est compris entre 70 et 250 mm, et le rayon des portées cylindriques de la plaque fémorale est compris entre 20 et 30 mm;
- la pièce fémorale, la plaque méniscale et le plateau tibial comportent tous une découpe dirigée vers la partie postérieure de la prothèse de genou ces découpes étant alignées afin de permettre le passage du ligament croisé antérieur;
- des protubérances formant des guides latéraux des parties latérales cylindriques de la plaque fémorale sont disposées sur les côtés intérieurs des portées cylindriques de la plaque méniscale;
- chaque protubérance se termine dans la zone médiane de plaque méniscale par un épaulement qui constitue un guide au rebord interne de la découpe de la pièce fémorale, lorsque la prothèse de genou est en position d'extension;
- la face externe du bouclier trochléen est dans sa partie médiane pourvue d'une rainure de guidage d'un coulisseau d'une prothèse rotulienne;
- le fond de la rainure de guidage est cylindrique et la face du coulisseau coopérant avec le fond de la rainure est également cylindrique avec le même rayon de courbure;
- la face opposée du coulisseau est plane et s'appuie contre l'une des faces d'une platine comportant une fente horizontale à travers laquelle s'étend une tige dont l'une des extremites est fixée au coulisseau et l'autre comporte une tête disposée de l'autre côte de la platine de manière à permettre un déplacement horizontal de la platine par rapport au coulisseau;
- la rotation de la platine est limitée par deux nervures parallèles disposées aux rebords supérieur et inférieur de la face postérieure de la platine;
- la tête de la tige est disposée dans une cavité délimitée par une bride faisant saillie de la face antérieure de la platine, cette bride étant pourvue d'un épaulement contre lequel est fixé une plaquette circulaire au centre de laquelle est fixée une vis, ces éléments constituant ensemble une pièce de fixation de l'os patellaire;
- la pièce fémorale, la plaque méniscale, le plateau tibial et le coulisseau de la prothèse rotulienne sont réalisés en alliage de titane, de chrome-cobalt, d'acier inoxydable.

L'invention sera maintenant décrite plus en détail à l'aide d'un exemple non limitatif en référence aux dessins annexés sur lesquels :
- La figure 1 est une vue éclatée en perspective des éléments constitutifs d'une prothèse de genou selon l'invention;
- La figure 2 est une vue frontale éclatée d'une prothèse de genou selon l'invention avec indication des endroits de fixation sur le fémur respectivement sur le tibia;
- La figure 3 est une vue de coupe correspondant à une coupe médiane de la figure 2;
- La figure 4 est une vue latérale schématique montrant la position relative, en flexion, des éléments constitutifs de la prothèse de genou selon l'invention;
- La figure 5 est une vue schématique latérale, partiellement découpée, de la prothèse de genou des figures 1 à 4 après sa mise en place et en position d'extension;
- La figure 6 est une vue schématique latérale, partiellement découpée, de la prothèse de genou des figures 1 à 4 après sa mise en place et en position de flexion; la figure montrant en outre les différents mouvements de rotation lors de la flexion;
- La figure 7 est une vue éclatée en perspective d'éléments formant une prothèse rotulienne suivant l'invention;
- La figure 8 est en vue en coupe d'une prothèse rotulienne suivant l'invention, après assemblage;
- Les figures 9 et 10 sont des vues schématiques dans le plan de la prothèse rotulienne illustrant les degrés de liberté de mouvement de celle-ci.

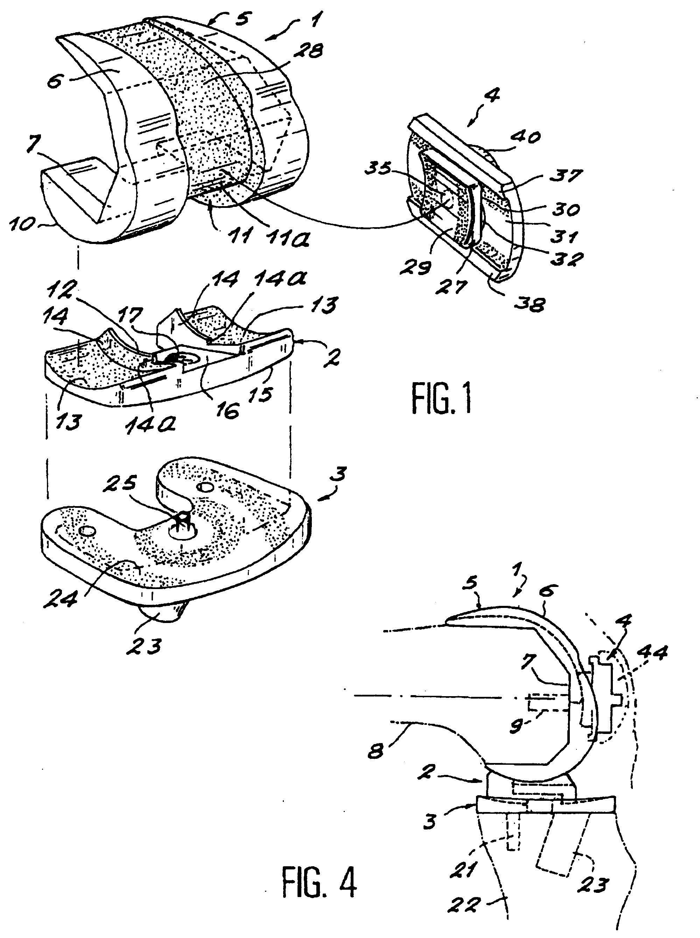

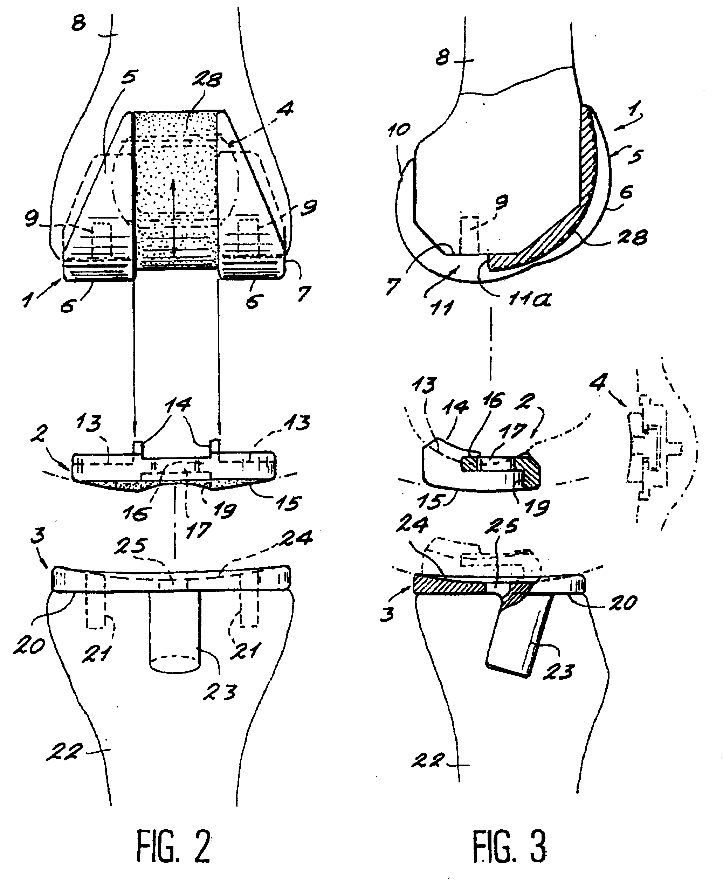

Les figures 1 à 4 montrent les éléments constitutifs de la prothèse de genou proprement dite.

Les éléments principaux de la prothèse de genou sont constitués par la pièce fémorale 1, la plaque intermédiaire méniscale 2 et le plateau tibial 3. Une prothèse rotulienne 4, qui sera décrite en détail plus tard, est associée à la prothèse de genou en coopérant avec la pièce fémorale 1.

La pièce fémorale 1 est composée d'une part d'un bouclier trochléen 5 présentant une face externe globalement cylindrique 6 et d'autre part d'une plaque fémorale 7 dont la partie postérieure est retournée vers le haut. Un siège de réception du fémur 8 est ainsi délimité par les parois internes du bouclier trochléen 5 et de la plaque fémorale 7. Le fémur 8 peut après insertion à force y être fixé à l'aide d'axes de scellement 9.

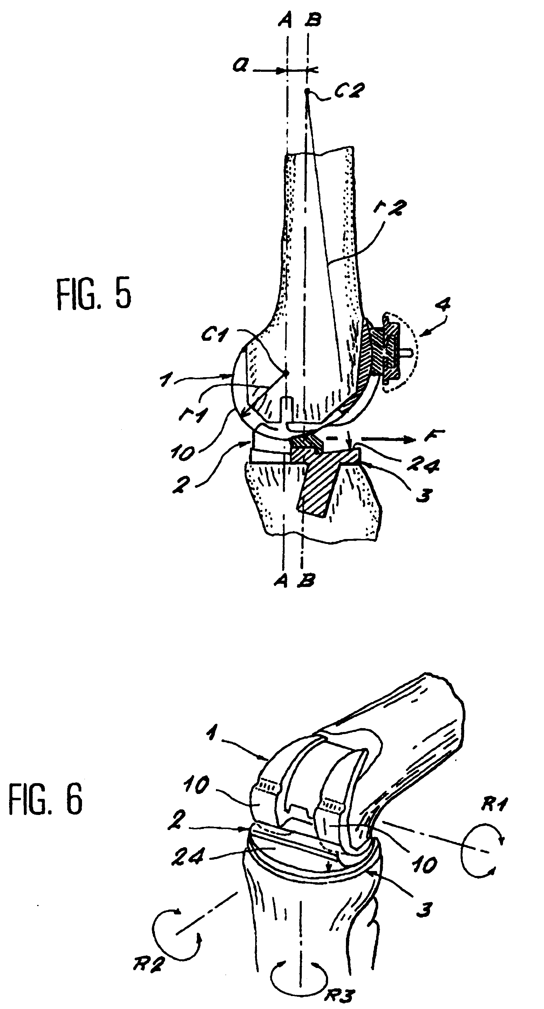

Les parties latérales de la plaque fémorale 7 forment des portées cylindriques 10 jouant le rôle de condyles. Le rayon r1 (voir figure 5) des cylindres est de préférence compris entre 20 et 30 mm. Ces portées cylindriques sont séparées l'une de l'autre par une découpe 11 effectuée dans la plaque fémorale de manière à former un U ouvert vers l'extérieur.

La plaque intermédiaire méniscale 2 est pourvue d'une échancrure 12 de manière à présenter une forme générale d'un U dont l'ouverture est dirigée ver la région postérieure de genou. L'échancrure 12 de la plaque méniscale se trouve en permanence en regard de la découpe 11 de la plaque fémorale 1 afin de permettre le passage du ligament croisé antérieur (non représenté).

La surface supérieure de la plaque méniscale 2 comporte deux portées concaves cylindriques 13 aménagées sur les branches du U et destinées à se trouver en regard des portées cylindriques 10 de la plaque fémorale 7. Le rayon de ces portées cylindriques est de préférence compris entre 20 et 30 mm et est toujours identique au rayon r1 des portées cylindriques 10 de la plaque fémorale 7 afin de permettre un contact optimal entre ces surfaces lors du fonctionnement.

Des protubérances 14 formant des guides latéraux des condyles de la plaque fémorale sont disposées sur les côtés intérieurs des portées cylindriques 13 de la plaque méniscale.

Chaque protubérance 14 se termine dans la zone médiane de la plaque méniscale 2 par un épaulement 14a qui constitue un guide au rebord interne 11a de la découpe 11 de la pièce fémorale 1, lorsque le genou vient en position d'extension.

La surface inférieure 15 de la plaque méniscale est convexe et sphérique. Le rayon de la sphère est de préférence compris entre 70 mm et 250 mm.

Dans cette forme de U, l'âme 16 est pourvu d'un trou traversant 17 pouvant, si besoin en est, recevoir un élément d'un système de stabilisation de la partie postérieure de la prothèse. Ce système de stabilisation n'est pas représenté sur les figures et ne sera pas décrit en détail puisqu'il est bien connu en soi et ne se rapporte pas directement à l'invention.

L'âme 16 est sur sa face inférieure pourvue d'une cavité 19 permettant la postéro-stabilisation par un plot 25 disposé dans sa partie médiane du plateau tibial 3.

Le plateau tibial 3 a également globalement la forme d'un U dont l'ouverture est dirigée vers l'arrière afin de permettre le passage du ligament croisé antérieur.

La face inférieure 20 du plateau tibial 3 est plane avec traitement de surface permettant la réhabilitation osseuse ou le scellement d'axes 21 dans le tibia 22. Il est en outre pourvu d'un plot de scellement central 23 avec possibilité de rallonge.

La face supérieure 24 du plateau tibial 3 est concave et sphérique. Elle constitue une portée coopérant avec la face inférieure sphérique de la plaque méniscale 2. Le rayon r2 (voir figure 5) de la sphère est également de préférence compris entre 70 mm et 250 mm mais est de toute manière identique au rayon de la plaque méniscale pour s'ajuster dans la surface sphérique de celle-ci.

Il est à noter que grâce à la forme sphérique 24 de la face supérieure du plateau tibial 3 s'ajustant à la forme sphérique 15 de la plaque méniscale 2 on obtient des degrés infinis de liberté de mouvement de la plaque méniscale 2 par rapport au plateau tibial 3.

Le principe de fonctionnement est basé sur la décomposition du mouvement de rotation-glissement en deux mouvements principaux: d'une part un mouvement de rotation R1 (voir figure 6) qui se fait par les condyles de la pièce fémorale 1 qui viennent tourner sur les portées cylindriques 13 du plateau méniscale 2 et d'autre part un glissement de la plaque méniscale 2 d'arrière en avant sur la portée sphérique du plateau tibial 3 dans le sens de la flèche F de la figure 5.

Ce glissement de la plaque méniscale 2 sur la portée sphérique du plateau tibial 3 est provoqué par le décalage, lorsque le genou est dans la position d'extension de la figure 5, d'un premier plan vertical A-A dans lequel est disposé le centre c1 de l'axe des faces cylindriques 6 formant condyles, par rapport à un deuxième plan vertical B-B dans lequel est disposé le centre c2 de la portée sphérique 24 du plateau tibial 3. Ce décalage a ainsi pour effet ledit glissement lors de la flexion du genou.

Grâce aux parties sphériques de la plaque méniscale 2 et du plateau tibial 3, l'auto-positionnement parfait des trois pièces 1, 2 et 3 s'effectuent, les degrés de liberté respectés évitant ainsi toutes contraintes aux scellements tout en respectant l'équilibre ligamentaire. La figure 6 illustrent en outre deux mouvements auxiliaires rendus possibles grâce à cette forme sphérique, à savoir un mouvement de rotation verticale R2 de la plaque méniscale 3 sur le plateau tibial 3 ainsi qu'un mouvement de rotation horizontale R3 de ces mêmes éléments l'un par rapport à l'autre.

Grâce à ce principe les condyles 6 de la pièce fémorale 1 sont en contact permanent avec les portées cylindrique 13 de la plaque méniscale 2 avec une surface de contact maximale de roulement entre les pièces 2 et 3 quelles que soient les positions, flexion ou extension, évitant ainsi toutes usures dues à des surcharges ponctuelles qui engendrent un poinçonnement et une usure accélérée de la prothèse.

Grâce à la forme du la plaque méniscale 2 et du plateau tibial 3 qui reçoivent la charge, ce dernier est toujours centré sur le centre de gravité, ceci permet de compenser d'une manière dynamique l'absence ou l'insuffisance du ligament croisé antérieur.

Dans les différentes positions du genou, flexion, extension, rotation, la transmission de la charge se fait par des surfaces maximales augmentant, par ce principe même, la longévité des matériaux.

La transmission de la charge se fait par des surfaces optimales, mais la longévité des surfaces frottantes en contact les unes avec les autres est davantage améliorée selon l'invention grâce à la mise en oeuvre d'une céramique sur toutes ces surfaces qui ainsi obtiennent la qualité des céramiques.

Il s'est avéré qu'une céramique tout simplement appliquée sur une surface frottante se brise facilement et l'invention propose pour cette raison d'encastrer chaque céramique dans la matière de la surface frottante. Ainsi sont obtenues des surfaces frottantes pratiquement incassables et extrêmement dures et résistantes à l'usure, ce qui assure la longévité des pièces bien au-delà de ce qui est actuellement connu pour de telles pièces.

Une telle céramique encastrée est ainsi appliquée sur toutes les surfaces frottantes, comme cela ressort des figures sur lesquelles ces céramiques sont schématiquement indiquées.

Les céramiques sont appliquées en couches, par exemple par plasma d'arc soufflé, dans des enfoncements effectués dans les surfaces frottantes et destinés à former des logements aux céramiques. Ce dispositif d'encastrement permet lors de la projection de mettre les couches de céramique en compression et par là même d'en améliorer les caractéristiques mécaniques: meilleure adhérence, plus grande tenue aux chocs et surtout une plus grande dureté.

Une autre caractéristique importante réside dans le fait que les céramiques sont réalisées avec une surépaisseur par rapport à leur support métallique, de sorte que les surfaces céramisées fonctionnant en vis-à-vis créent des canaux de lubrification apportant aux surfaces de glissement le liquide synoviale, permettant ainsi une parfaite lubrification de l'articulation.

La céramique peut être constituée de par exemple du Al203, Al203-Ti02, Zr02-Y203 ou Cr203 allié à des métaux Cr ou Ti. Chaque céramique encastrée a une épaisseur de préférence comprise entre 0,1 et 0,6 mm.

Les céramiques sont appliquées sur les parties condyliennes 10 et la rainure 28 de guidage rotulienne de la pièce fémorale 1, les portées cylindriques 13 et les faces des protubérances 14 de la plaque méniscale 2 tournées vers ces portées, et sur la face sphérique 15 de la plaque méniscale 2 en contact avec la portée sphérique 24 du plateau tibial 3, ainsi que sur cette dernière.

Une telle couche est également appliquée sur la face postérieure 29 du coulisseau coopérant avec le fond de la rainure 28 de la pièce fémorale 1 et avantageusement aussi sur la face opposée en contact avec la face postérieure de la platine 31, cette face étant dans ce cas également pourvue d'une couche de céramique.

Il suffit qu'une seule de deux surfaces frottantes en contact l'une avec l'autre soit entièrement couverte d'une céramique, l'autre surface pouvant l'être partiellement. Il est ainsi montrée à la figure 1 que la face supérieure 24 du plateau tibial 3 est partiellement recouverte par des céramiques en forme de bandes latérales incurvées et une bande centrale circulaire. De la même manière dans la prothèse rotulienne, la surface de la platine 31 coopérant avec la face plane 30 du coulisseau 27 comporte deux bandes latérales rectilignes. La face cylindrique 29 du coulisseau 27 en contact avec la rainure de guidage 28 comporte également des bandes latérales, alors que le fond est les côtés de la rainure 28 sont entièrement recouvertes d'une céramique encastrée.

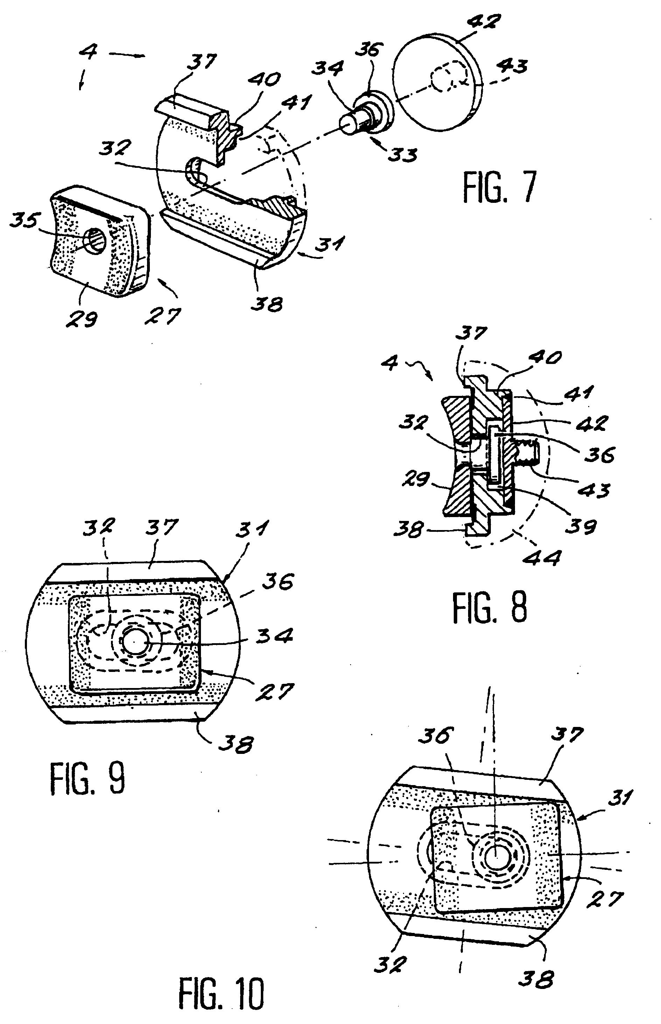

La prothèse de genou qui vient d'être décrite peut avantageusement être associée à une prothèse rotulienne illustrée plus en détail sur les figures 7 à 10.

La prothèse rotulienne 4 comporte un coulisseau 27 constitué par une plaque globalement rectangulaire qui peut coulisser le long d'une rainure centrale de guidage 28 ménagée sur la face externe du bouclier trochléen 5 de manière à permettre le déplacement par rotation de la pièce fémorale 1 par rapport à l'ensemble de la prothèse rotulienne lors de la flexion et de l'extension du genou.

Le fond de la rainure de guidage 28 est cylindrique et la face postérieure 29 du coulisseau coopérant avec le fond de la rainure 28 est également cylindrique avec le même rayon de courbure qui par ailleurs correspond au rayon de la face externe du bouclier de sorte que la profondeur de la rainure soit constante pour bien assurer le guidage de la prothèse rotulienne lors de la flexion et de l'extension du genou.

La face antérieure opposée 30 du coulisseau 27 est plane et s'appuie contre la face postérieure d'une platine 31 aux extrémités horizontales arrondies. La platine 31 comporte une fente horizontale 32 à travers laquelle s'étend un rivet 33 dont la tige 34 est rivetée dans un trou traversant 35 ménagé dans le coulisseau 27 et dont la tête 36 se trouve disposée de l'autre côté de la platine, à la face antérieure de celle-ci.

Le diamètre de la tige 34 du rivet 33 est légèrement inférieur à la largeur de la fente 32 afin de permettre à la tige 34 de coulisser le long de la fente.

La rotation de la platine 31 est limitée par deux nervures parallèles 37, 38 disposées aux rebords supérieur et inférieur de la face postérieure de la platine. Lors de la rotation de la platine 31, les nervures 37, 38 viennent buter (voir figure 10) contre les rebords du coulisseau 27 qui lui ne peut pas tourner puisqu'il est maintenu horizontalement par les parois latérales de la rainure de guidage 28 de la pièce fémorale 1.

La tête 36 du rivet 33 se trouve dans une cavité 39 délimitée par une bride 40 faisant saillie à la face opposée de la platine 31. Cette bride 40 est pourvue d'un épaulement 41 contre lequel est fixé une plaquette circulaire 42. Au centre de cette plaquette est fixée une vis 43.

L'ensemble formé par la platine 31, la plaquette 42 et la vis 43 constitue une pièce de fixation de l'os patellaire 44.

La pièce fémorale 1, la plaque méniscale 2, le plateau tibial 3 et le coulisseau 27 de la prothèse rotulienne 4 sont avantageusement réalisées en alliage de titane, de chrome-cobalt, d'acier inoxydable.

Lors de la flexion par la rotation de la pièce fémorale 1, le coulisseau 27 de la prothèse rotulienne reste à peu près au même niveau mais effectue un mouvement relatif par rapport à la pièce fémorale 1 tout en étant déplacée le long de la rainure de guidage cylindrique 28 de celle-ci.

Grâce à l'invention est ainsi obtenue une prothèse de genou qui propose une solution efficace au problème associé à l'usure des pièces en contact, contrairement aux prothèses connues qui sont toutes à cinétique de destruction relativement rapide.

La prothèse suivant l'invention permet l'auto-positionnement de l'ensemble des trois pièces et compense automatiquement les défauts de coupes osseuses. On évite ainsi une surcharge pondérale très importante condyles-pièces mobiles, ce qui contribue également à une augmentation de la longévité de la prothèse. Il est aussi à noter que les céramiques sont indispensables pour le bon fonctionnement de l'articulation.

## Revendications

1. Prothèse de genou comprenant une pièce fémorale (1) reliée à un plateau tibial (3) par une plaque méniscale intermédiaire (2), la pièce fémorale (1) étant composée d'un bouclier trochléen (5) et d'une plaque fémorale (7) reliée à la partie inférieure du bouclier trochléen (5) et s'étendant vers l'arrière, les faces externes des parties latérales (10) de la pièce fémorale (1) étant cylindriques et formant des condyles coopérant avec des portées de guidage cylindriques (13) ménagées dans la face supérieure de la plaque méniscale (2) , la face inférieure (15) de celle-ci étant convexe afin de coopérer avec la face supérieure concave (24) du plateau tibial (3), caractérisée en ce que toutes les surfaces en contact de frottement les unes avec les autres sont au moins partiellement pourvues d'une céramique encastrée dans lesdites surfaces de frottement.

2. Prothèse de genou selon la revendication 1, caractérisée en ce que les céramiques font légèrement saillie par rapport aux surfaces de frottement dans lesquelles elles se trouvent encastrées.

3. Prothèse de genou selon la revendication 1 ou 2, caractérisée en ce que l'épaisseur de la céramique encastrée est comprise entre 0,1 et 0,6 mm.

4. Prothèse de genou selon l'une quelconque des revendications précédentes, caractérisée en ce que l'une de deux surfaces de frottement coopérant l'une avec l'autre est partiellement recouverte par des céramiques encastrées sous forme de bandes.

5. Prothèse de genour selon l'une quelconque des revendications précédentes, caractérisée en ce que les faces convexe (15) et concave (24) de la plaque méniscale (2) et du plateau tibial (3), respectivement, sont sphériques, et en ce que le centre (c1) des portées cylindriques (10) de la pièce fémorale (1) est disposé, lorsque le genou est en position d'extension verticale, dans un plan vertical (A-A) décalé par rapport à un plan vertical (B-B) passant par le centre (c2) de la portée sphérique (24) du plateau tibial (3), de manière à provoquer un glissement de la plaque méniscale (2) sur le plateau tibial (3) d'arrière en avant lors de la flexion du genou.

6. Prothèse de genou selon la revendication 5, caractérisée en ce que le rayon (r2) de la sphère (15, 24) est compris entre 70 et 250 mm, et en ce que le rayon (r1) des portées cylindriques 10 de la plaque fémorale 7 est compris entre 20 et 30 mm.

7. Prothèse de genou selon l'une quelconque des revendications précedentes, caracterisee en ce que la pièce fémorale (1), la plaque miéniscale (2) et le plateau tibial (3) comportent tous une découpe (11; 12) dirigée vers la partie postérieure de la prothèse de genou ces découpes étant alignées afin de permettre le passage du ligament croisé antérieur.

8. Prothèse de genou selon l'une quelconque des revendications précédentes, caractérisée en ce que des protubérances (14) formant des guides latéraux des parties latérales cylindriques (6) de la plaque fémorale (1) sont disposées sur les côtés intérieurs des portées cylindriques (13) de la plaque méniscale (2).

9. Prothèse de genou selon la revendication 8, caractérisée ce que chaque protubérance (14) se termine dans la zone médiane de plaque méniscale (2) par un épaulement (14a) qui constitue un guide au rebord interne (11a) de la découpe (11) de la pièce fémorale (1), lorsque la prothèse de genou est en position d'extension.

10. Prothèse de genou selon l'une quelconque des revendications précédentes, caractérisée en ce que la face externe du bouclier trochléen (5) est dans sa partie médiane pourvue d'une rainure de guidage (28) d'un coulisseau (27) d'une prothèse rotulienne (4).

11. Prothèse de genou selon la revendication 10, caractérisée en ce que le fond de la rainure de guidage (28) est cylindrique et la face du coulisseau (27) coopérant avec le fond de la rainure est également cylindrique avec le même rayon de courbure.

12. Prothèse de genou selon la revendication 10 ou 11, caractérisée en ce que la face opposée du coulisseau (27) est plane et s'appuie contre l'une des faces d'une platine comportant une fente horizontale (32) à travers laquelle s'étend une tige (34) dont l'une des extrémités est fixée au coulisseau (27) et l'autre comporte une tête (36) disposée de l'autre côté de la platine (31) de manière à permettre un déplacement horizontal de la platine (31) par rapport au coulisseau (27).

13. Prothèse de genou selon la revendication 12, caractérisée en ce que la rotation de la platine (31) est limitée par deux nervures (37, 38) parallèles disposées aux rebords supérieur et inférieur de la face postérieure de la platine.

14. Prothèse de genou selon la revendication 12 ou 13, caractérisée en ce que la tête (36) de la tige (34) est disposée dans une cavité (39) délimitée par une bride (40) faisant saillie de la face antérieure de la platine (31), cette bride (40) étant pourvue d'un épaulement (41) contre lequel est fixé une plaquette circulaire (42) au centre de laquelle est fixée une vis (43), ces éléments constituant ensemble une pièce de fixation de l'os patellaire.

15. Prothèse de genou selon l'une quelconque des revendications précédentes, caractérisée en ce que la pièce fémorale (1), la plaque méniscale (2), le plateau tibial (3) et le coulisseau (27) de la prothèse rotulienne (4) sont réalisés en alliage de titane, de chrome-cobalt, d'acier inoxydable.
